# EUROPEAN PATENT APPLICATION

(11) **EP 1 400 920 A2**
(43) Date of publication of application: **24.03.2004**
(21) Application number: 03005341.7
(22) Date of filing: 12.03.2003
(51) Int. Cl.: G06T 5/00

(54) **Image processing device and ultrasonic diagnostic device**

(30) Priority: 14.03.2002 JP 2002070562
(71) Applicant: MATSUSHITA ELECTRIC INDUSTRIAL CO., LTD., Kadoma-shi, Osaka 571-8501 (JP)
(72) Inventor: Yamauchi, Masaki, Ibaraki-shi, Osaka 567-0863 (JP)
(74) Representative: Balsters, Robert

(57) **Abstract**

An area dividing unit 103 divides an ultrasound image into sub-areas in accordance with an initial contour. An evaluation value calculating unit 104 calculates evaluation values on the basis of information which includes, for example, brightness value-related information (e.g. contrast distribution) and position-related information (e.g. a distance from a reference point) of each of the sub-areas, and shape-related information (e.g. presence/absence of an edge). An area selecting unit 106 selects one or more sub-areas according to the calculated evaluation values. An each area processing unit 105 performs image processing appropriate to the selected sub-areas. An image reconstructing unit 107 reconstructs the ultrasound image using the sub-areas for which image processing has been performed.

## Description

### BACKGROUND OF THE INVENTION

### (1) Field of the Invention

This invention relates to an ultrasonic diagnostic device that generates an ultrasound image used in such a field as clinical medicine, and to an image processing device that processes an image displayed on various kinds of image-related devices, mobile phones and the like, and particularly to a technique for improving image quality such as contour extraction performed for the above-mentioned images.

### (2) Description of the Related Art

Image processing is sometimes performed by ultrasonic diagnostic devices, a wide range of image-related devices and the like for a specific object in an image (e.g. a soft tissue of a living body, a face) so as to extract its contour.

Ultrasonic diagnostic devices have been widely used as indispensable devices in such a filed as clinical medicine, since they are capable of obtaining a two-dimensional (2D) image of an object to be examined without invasion as well as offering a high level of safety to a living body. The same is also applicable to other devices utilizing an ultrasonic wave employed in other fields.

Generally, an ultrasonic diagnostic device receives an echo obtained when ultrasound emitted from an ultrasonic probe is partially reflected on reflection points and surfaces of tissue of an object of a living body to be examined, and generates an ultrasound image based on the received echo of the examined object. Since this reflected wave (ultrasonic echo) is feeble compared with the emitted ultrasound, amplification process (gain process) is performed for such reflected wave when a brightness signal is generated for displaying an image. Amplification (gain) control, i.e. brightness control for image quality, is conventionally conducted through a method known as STC (Sensitivity Time Control) in which a plurality of sliders (e.g. 16 sliders) classified according to the depth level of an examined object are operated for making control. (Note that processing utilizing a logarithmic amplifier is used in some cases.)

As described above, amplification process performed by a conventional ultrasonic diagnostic device is intended to control image quality by manually controlling contrast and dynamic range of an ultrasound image.

Meanwhile, by calculating values including the area/volume of a fetus and internal/circularly organs as well as the amount of their variations on the basis of an ultrasound image, it is possible to improve the quality of screening and scanning performed by an ultrasonic diagnostic device. In so doing, how a contour or a boundary of an organ and other examined objects used for calculating their area and volume is extracted, is of great importance.

However, methods including STC in which contrast or others of an examined object is manually controlled involve complicated processing as well as requiring some skills. Furthermore, when a contour or the like of an examined object is extracted only by tracing it manually, it always requires an accurate tracing by the use of such a tool as a pointing device. Therefore, a great deal of labor is required for an operator who traces the contour or the like of the examined object. Against this backdrop, a number of methods have been proposed for automatic image correction and contour/boundary extraction performed on an ultrasound image.

One example is a "method for automatic image quality correction" disclosed in Japanese Laid-open Patent Application No. 2002-209891, in which gain control is automatically performed on the basis of the characteristics of an ultrasound image (e.g. a brightness signal of an ultrasound image represented by the Gaussian distribution shows a steep distribution, and its effective dynamic range is narrow). With this method, gain control is performed by measuring the distribution of brightness values for the whole image in a uniform manner.

Another characteristic of an ultrasound image is that a part of the image is often unclear or does not properly appear on the image. However, with the above-mentioned method in which a uniform processing is performed for the whole image, there occurs a possibility that the image quality of an ultrasound image that is partially unclear or does not properly appear on the image cannot be sufficiently improved.

The same is also true of contour and boundary extraction methods. Conventional methods for extracting contours and boundaries are effective on the assumption that a contour of a specific object shows up clearly in an ultrasound image. The same can be also said to semiautomatic extraction methods in which a contour/boundary of an object is traced after it is given in advance an initial contour by a human hand. For example, in an "ultrasonic image diagnostic device" disclosed in Japanese Laid-open Patent Application No. H11-164834, a contour or the like of a target tissue is roughly traced by hand using a mouse or the like first, so as to extract a contour or the like serving as a guide, and then the start point is set for extracting the contour or the like. In this case, scan lines radiate in all directions from such start point. Then, based on intersection points of such lines and the above contour or the like extracted by hand, an area to be detected is determined. Subsequently, binarization is performed for image data within such detection area of the ultrasound image using a threshold value so as to detect a position on the contour or the like to be corrected. When the position on such contour or the like is detected, a further correction is made to the boundary of the contour or the like traced by hand so that a correct contour or the like can be obtained.

If one is skilled with this technique, it is possible to extract a contour or the like more speedily than a method with which a contour or the like is extracted only by a human hand. However, the problem is that this method is not fully automated. Moreover, this method is not intended for calibrating a contour or the like when it is inappropriately extracted. Consequently, a result of contour extraction varies depending on a threshold value to be set which is a prerequisite for binarization to be performed. As for an area which does not have a clear contour in the first place, there is no solution at all.

As described above, if a part of an ultrasound image is unclear or does not properly appear on the image, there occurs a possibility that conventional image control methods and contour extraction methods do not serve part of their purposes (or no purposes at all in some cases).

Meanwhile, an image of a human figure or a face (to be referred to as "human image" hereinafter) taken by a variety of image-related devices capable of taking pictures (mobile phones, PDAs and the like are especially used) are often generated nowadays, but a person who takes a picture sometimes wishes to perform image processing for the image s/he desires by extracting the contour of a face or others in the image. To be more specific, it is sometimes witnessed in a human image that a contour of a person (especially its face) becomes blurred depending on the background of a place where the image is taken or due to such an atmosphere as steam coming up around such place. In such cases, it is possible to perform image processing for clarifying the contour of the person without artificiality.

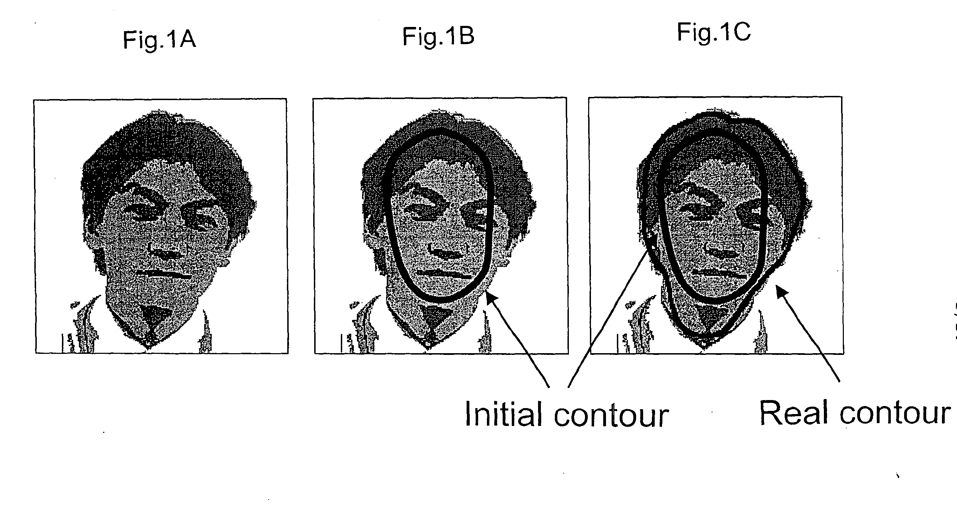

Figs.1A∼1C are diagrams showing an example case where contour extraction performed for a human image is successful by the use of a conventional image-related device.

Fig.1A is an original image taken by a mobile phone. As illustrated in Fig.1A, only a human face is shown in the original image. The following gives an explanation for the case where contour extraction is performed for such original image. As a contour extraction method, there is a method disclosed in Japanese Laid-open Patent Application No. 2002-224116 in which a contour of an object is extracted through two steps. According to this method, an initial contour is specified first (as illustrated in Fig.1B) and then a more precise contour is extracted (as illustrated in Fig.1C).

However, if there exists a part in the original image that hinders contour extraction, an expected contour might not be extracted.

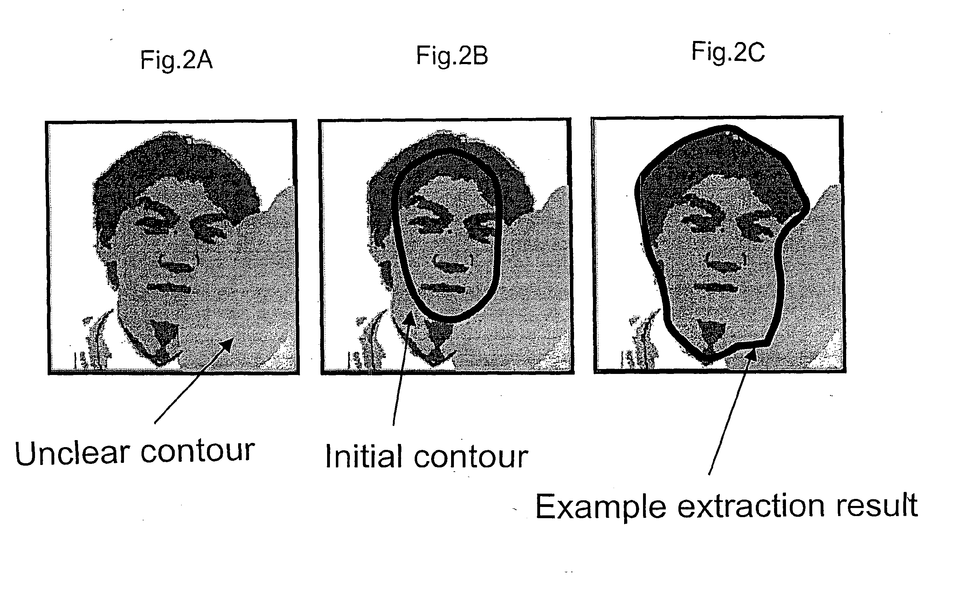

Figs.2A∼2C are diagrams showing an example case where contour extraction performed for a human image ends in failure by the use of a conventional image-related device.

Fig.2A is an original image equivalent to that shown in Fig.1A, but since there is a part that hinders contour extraction for the lower left-hand part of the image (e.g. a part where water vapor appears), Fig.2A is different from Fig.1A in that a part of the face contour is blurred. When the same contour extraction method as used for the original image in Figs.1A∼1C is employed for the original image in Fig.2A (Fig.2B illustrates the case where an initial contour is specified), processing intended for extracting a more precise contour results in a contour different from the real one. As described above, if there exists a part in the original image that hinders contour extraction, there may occur a problem that an expected contour cannot be extracted.

### SUMMARY OF THE INVENTION

The present invention, which is made in view of the above problems, aims at providing a variety of image processing methods to be employed according to local characteristics of an ultrasound image, as wells as providing automatic correction and contour extraction methods for images through such image processing methods.

The image processing device and the ultrasonic diagnostic device according to the present invention divide an image into sub-areas and perform image processing appropriate to each of such sub-areas. Accordingly, it is possible for the present invention to overcome drawbacks of a conventional device such as that automatic image quality control does not function due to an image having a part which does not appear properly or having an unclear edge because contrast is low in some parts. Moreover, it is also possible for the present invention to overcome such a drawback of a conventional device as that contour/boundary extraction methods do not function properly due to the above reasons.

To put it another way, the above-mentioned drawbacks stem from the fact that conventional contour/boundary extraction methods are effective on the assumption that a contour is always extracted clearly. However, it is possible with the present invention to improve the clarity of an image which is partly low-contrasted.

In order to achieve the above objects, the image processing device according to the present invention is an image processing device comprising: an image acquiring unit operable to acquire image data; an area dividing unit operable to divide an image represented by the acquired image data into a plurality of sub-areas; an area selecting unit operable to make a selection of one or more of the sub-areas; and an each area processing unit operable to perform image processing for each of said one or more selected sub-areas.

Moreover, in order to achieve the above objects, the ultrasonic diagnostic device according to the present invention is an ultrasonic diagnostic device that displays an ultrasound image of an object subject to examination generated on the basis of a reflection of ultrasound and that comprises: an image acquiring unit operable to acquire image data; an area dividing unit operable to divide an ultrasound image represented by the acquired image data into a plurality of sub-areas; an area selecting unit operable to make a selection of one or more of the sub-areas; an each area processing unit operable to perform specific image processing for each of said one or more selected sub-areas; and a displaying unit operable to display an image of said one or more selected sub-areas for which the image processing is performed.

Note that, in order to achieve the above objects, the present invention may be implemented as a program which includes the characteristic units of the image processing device and the ultrasonic diagnostic device as its steps. Furthermore, it is also possible for such program not only to be stored in a ROM and the like in the image processing device and the ultrasonic diagnostic device but also be distributed through storage media such as CD-ROM, or over transmission media such as communications network.

Japanese patent application No. 2002-070562 filed March 14 2002, is incorporated herein by reference.

### BRIEF DESCRIPTION OF THE DRAWINGS

These and other subjects, advantages and features of the invention will become apparent from the following description thereof taken in conjunction with the accompanying drawings that illustrate a specific embodiment of the invention. In the Drawings:
Fig.1A is a diagram showing an example original image taken by a conventional mobile phone.
Fig.1B is diagram showing the original image of Fig.1A for which an initial contour has been identified.
Fig.1C is a diagram showing an example case where a more precise contour is successfully extracted on the basis of the original image of Fig.1B.
Fig.2A is a diagram showing another example original image taken by a conventional mobile phone.
Fig.2B is diagram showing the original image of Fig.2A for which an initial contour has been identified.
Fig.2C is a diagram showing an example case where a more precise contour is unsuccessfully extracted on the basis of the original image of Fig.2B.
Fig.3 is a block diagram showing an overview of a functional configuration of an ultrasonic diagnostic device according to the first embodiment.
Fig.4 is a diagram showing a detailed functional configuration of the image processing unit in Fig.3.
Fig.5 is a diagram explaining a method in which an initial contour of an object is specified through automatic extraction or an operator's operation, and then an ultrasound image is divided from a gravity center of such initial contour in a radial pattern.
Fig.6 is a diagram explaining a variation of the method presented in Fig.5.
Fig.7 is a diagram explaining a method in which a boundary having a certain number of pixels in the outward direction around the specified initial contour is drawn, and then a doughnut-shaped area in between the initial contour and such boundary is divided in a radial pattern at a specified angle.
Fig.8 is a diagram explaining a variation of the method presented in Fig.7.
Fig.9 is a diagram explaining a method in which an ultrasound image is divided into "N" equal parts in the directions of the vertical axis and the horizontal axis respectively.
Fig.10 is a diagram showing an example distribution of brightness values of sub-areas of an ultrasound image.
Fig.11A is a diagram showing input brightness values and output brightness values at the time of binarization process.
Fig.11B is a diagram showing a relationship between input brightness values and output brightness values at the time of contrast control process and bias control process.
Fig.12 is a diagram showing an example method for transforming a brightness value distribution.
Fig.13 is a simplified diagram showing an ultrasound image before image processing is performed by the each area processing unit.
Fig.14 is a simplified diagram showing an ultrasound image after image processing is performed by the each area processing unit.
Fig.15 is a flowchart showing an example overall flow of processing performed by the ultrasonic diagnostic device.
Fig.16 is a flowchart showing an example of "Area division processing" illustrated in Fig.14.
Fig.17 is a flowchart showing an example of "Evaluation value calculation processing" illustrated in Fig.14.
Fig.18 is a flowchart showing an example of "Area-by-area processing" illustrated in Fig.14.
Fig.19 is a flowchart showing an example of "Image reconstruction processing" illustrated in Fig.14.
Fig.20 is a block diagram showing an overview of a functional configuration of an image processing device according to the second embodiment.
Fig.21A is an example original image taken by a mobile phone.
Fig.21B is a diagram showing the original image of Fig.21A for which an initial contour has been specified.
Fig.21C is a diagram showing the image of Fig.21B for which area division has been performed.
Fig.22 A is a diagram showing that sub-areas are selected from the image divided in Fig.21C.
Fig.22B is a diagram showing that image processing is performed for the sub-areas selected in Fig.22A.
Fig.23A is a diagram showing that an initial contour is specified in the image of Fig.22B for which image processing has been performed.
Fig.23B is a diagram showing that a precise contour is extracted on the basis of the image of Fig.23A.
Fig.24A is a diagram showing an example original image taken by a mobile phone.
Fig.24B is a diagram showing the original image of Fig.24A for which a precise contour has been extracted.
Fig.24C is a diagram showing an example of how the extracted face contour is made "smaller".
Fig.24D is a diagram showing that the face is made "slimmer" and "smaller" on the basis of the extracted face contour.
Fig.25 is a diagram showing chromakey is performed by overlaying the face specified by the contour extraction on another image.
Fig.26 is a flowchart showing an example overall flow of the image processing device.
Fig.27A is a diagram showing a reference point specified on a contour line.
Fig.27B is a diagram showing an area tile being defined with the reference point in Fig.27A as the center.
Fig.27C is a diagram showing area tiles being defined for the entire image along the contour line, on the basis of the area tile defined in Fig.27B.
Fig.28A is a diagram showing the image being divided according to the area tiles which have been defined on the basis of the contour line, the circumscribed rectangle, the external rectangle, and the internal rectangle.
Fig.28B is a diagram showing the image being divided according to the area tiles which have been defined on the basis of the contour line, the external rectangle, and the internal rectangle.

### DESCRIPTION OF THE PREFERRED EMBODIMENTS

The following explains preferred embodiments according to the present invention with reference to the figures.

### (First Embodiment)

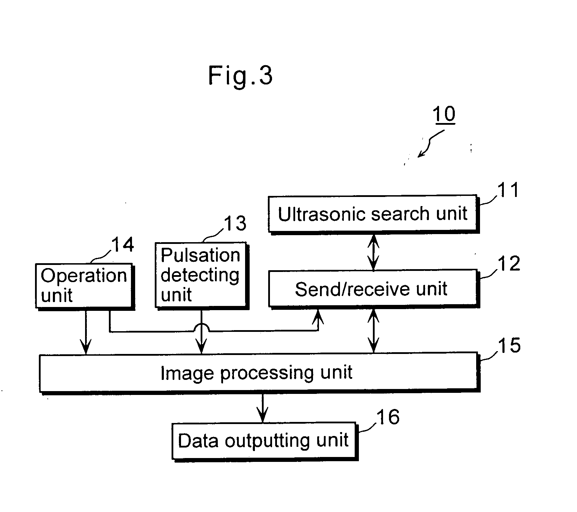

Fig.3 is a block diagram showing an overview of a functional configuration of an ultrasonic diagnostic device 10 according to the present embodiment, which is one of the image processing devices according to the present invention. The ultrasonic diagnostic device 10 is capable of performing case-by-case processing for improving image quality even when a part of an ultrasound image is unclear or blurred. Such ultrasonic diagnostic device 10 is comprised of an ultrasonic search unit 11, a send/receive unit 12, a pulsation detecting unit 13, an operation unit 14, an image processing unit 15, and a data outputting unit 16.

The ultrasonic search unit 11, which is generally called a probe, may be a probe that performs electronic scan based on the phased array method. The ultrasonic search unit 11 emits ultrasound (e.g. ultrasonic pulse) on the basis of a control signal sent by the send/receive unit 12. Furthermore, the ultrasonic search unit 11 converts the ultrasound (to be referred to as ultrasonic echo hereinafter) reflected from inside the living body of a subject into an electric signal, and sends it to the send/receive unit 12.

The send/receive unit 12, which includes, for example, a CPU, a ROM, a RAM, or the like, has an overall control of the ultrasonic diagnostic device 10 as well as a function to send/receive ultrasound. Other constituent elements of the send/receive unit 12 include a sender/beam former for having the ultrasonic search unit 11 generate ultrasound and a receiver/beam former for receiving an electric signal sent from the ultrasonic search unit 11 that has detected an ultrasonic echo. Subsequently, the send/receive unit 12 performs processing such as amplification for the electric signal sent from the ultrasonic search unit 11, and sends such processed electric signal to the image processing unit 15. Furthermore, the send/receive unit 12 accepts an instruction from an operator via the operation unit 14.

The pulsation detecting unit 13, an example of which is a pulsation sensor, converts the detected pulsation of the subject into an electric signal, and sends it to the image processing unit 15.

The operation unit 14, which includes a switch, a touch panel and others, accepts from the operator operations performed on them, and sends to the send/receive unit 12 and the image processing unit 15 a control signal or the like corresponding to such operations.

The image processing unit 15 generates image data of an ultrasound image based on the electric signal sent from the send/receive unit 12. Then, the image processing unit 15 divides the generated ultrasound image into sub-areas, and performs image processing for each sub-area. Furthermore, the image processing unit 15 reconstructs the ultrasound image on the basis of the processed image data, and sends the resulting image data to the data outputting unit 16.

The data outputting unit 16, which is made up of a graphic accelerator, a scan converter and others, is capable of receiving image data of the ultrasound image reconstructed by the image processing unit 15 (e.g. B-mode ultrasound image) so as to show such image data on a liquid crystal display or the like serving as an observation monitor.

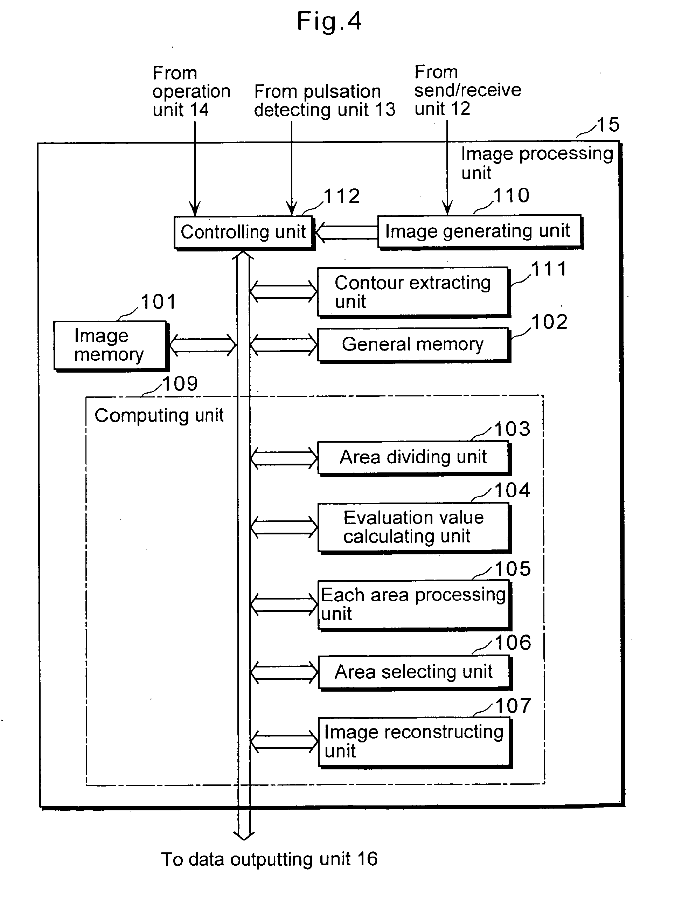

Fig.4 is a block diagram showing a detailed functional configuration of the image processing unit 15 illustrated in Fig.3. Such image processing unit 15 is comprised of an image generating unit 110, a contour extracting unit 111, a controlling unit 112, an image memory 101, a general memory 102, and a computing unit 109. The computing unit 109, which features the present invention, is embodied by hardware like a specialized processor or the like, or software. Such computing unit 109 is made up of an area dividing unit 103, an evaluation value calculating unit 104, an each area processing unit 105, an area selecting unit 106, and an image reconstructing unit 107.

The image generating unit 110 generates image data by performing A/D conversion or the like for an electric signal sent from the send/receive unit 12. Furthermore, the image generating unit 110 sends such generated image data to the controlling unit 112.

Image data here refers to 2D brightness data or the like that is generated each time scanning is performed by the ultrasonic search unit 11 and that is to be displayed in B-mode and the like.

The contour extracting unit 111 extracts a contour of such an object as the left ventricle (LV) of a heart on the basis of image data stored in the image memory 101, and generates contour data. Note that details of a method for extracting a contour based on image data are described in Japanese Laid-open Patent Application No. 2002-224116. To summarize this method, a rough initial contour is extracted by performing "binarization" and "degeneracy" for an ultrasound image of a target object. Then, after a dynamic contour model (SNAKES) is applied to the initial contour, convergent calculation is performed for such initial contour so as to specify a precise contour in the end. Contour data here refers to data including coordinate (X axis and Y axis) data of a plurality of pixels making up a contour line of an examined object that is extracted on the basis of image data in one frame.

The controlling unit 112, an example of which is a microcomputer having a ROM, a RAM and others, gives instructions mainly to the units in the image processing unit 15 to have them execute their own processing, and controls timing of such processing.

At the instruction of the controlling unit 112, the image memory 101 (e.g. a RAM) stores the image data of the ultrasound image generated by the image generating unit 110 and image data for which image processing has been performed by the below-described each area processing unit 105.

At the instruction of the controlling unit 112, the general memory 102 (e.g. a RAM) stores data other than image data of the ultrasound image generated by the image generating unit 110 (i.e. data stored in the image memory 101) such as data related to area division, data associated with a contour, data related to evaluation value calculation, and data related image processing).

The area dividing unit 103 divides the ultrasound image generated by the image generating unit 110 into a plurality of sub-areas. The following are example methods for area division:
① Specify an initial contour of a target object through automatic extraction or an operation of the operator, and then divide the ultrasound image in a radial pattern from the gravity center of the ultrasound image as the starting point;
② Draw a boundary having a certain number of pixels in the outward direction around the initial contour which has been specified using the above method ①, and then divide a doughnut-shaped area in between the initial contour and such boundary in a radial pattern at a specified angle (e.g. π/4); and
③ Divide the ultrasound image into "N" equal sub-areas (e.g. into quarters) in the directions of the vertical axis and the horizontal axis respectively.

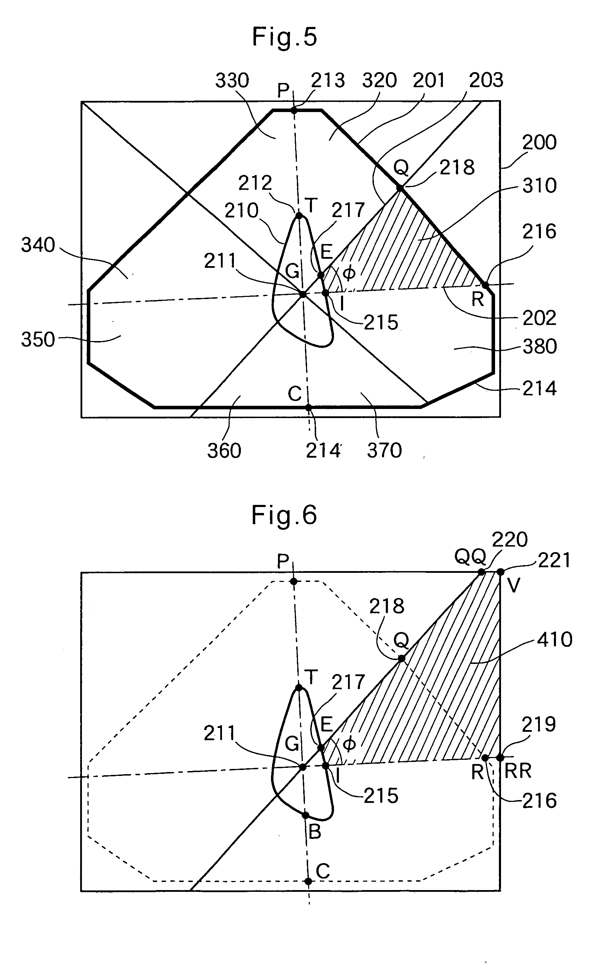

Fig.5 explains an example of the method ① described above. In Fig.5, a rectangular frame 200 indicates the outer edge of an area which can be displayed on the observation monitor of the data outputting unit 16, while a fan-shaped area enclosed by a bold line 201 indicates an area in the ultrasound image to be actually displayed on the observation monitor. Fig.5 shows eight divided sub-areas 310∼380.

The following explains the procedure to be performed until the area dividing unit 103 determines the sub-area 310.

First, an initial contour 210 is specified through automatic extraction or an operation of the operator, and then a gravity center G211 of such initial contour 210 is calculated. Then, a top T212 serving as a reference point on the initial contour 210 (i.e. the point indicating the biggest Y axis value in the initial contour 210) is identified, and then a point P213 and a point C214 are determined which intersect with the bold line 201 when a straight line between the gravity center G211 and the top T212 is extended.

Next, two straight lines 202 and 203 are determined that form angles of (π/2) and (-π/4) between the straight line PC connecting the point P213 and the point C214. Then, points at which such two straight lines 202 and 203 intersect with the initial contour 210 are defined respectively as a point I215 and a point E217, and points at which such two straight lines 202 and 203 intersect with the bold line 201 are defined respectively as a point R216 and a point Q218.

A closed area to be formed by connecting the point 1215, the point R216, the point Q218 and the point E217 (the diagonally shaded area in Fig.5) indicates the sub-area 310, which is one of the divided eight sub-areas. The other sub-areas 320∼380 are determined in the same manner.

Fig.6 explains a variation of the method ① described above. While Fig.5 illustrates the case where the area between the initial contour 210 and the bold line 201 is the target of division (only the area to be actually displayed on the monitor is the target), Fig.6 illustrates the case where a target area to be divided is extended to the rectangular frame 200. Accordingly, a disclosed area to be formed by connecting the point I215, a point RR 219, a point V221, a point QQ 220, and the point E217 (the diagonally shaded area in Fig.6) indicates a determined sub-area 410 in this case.

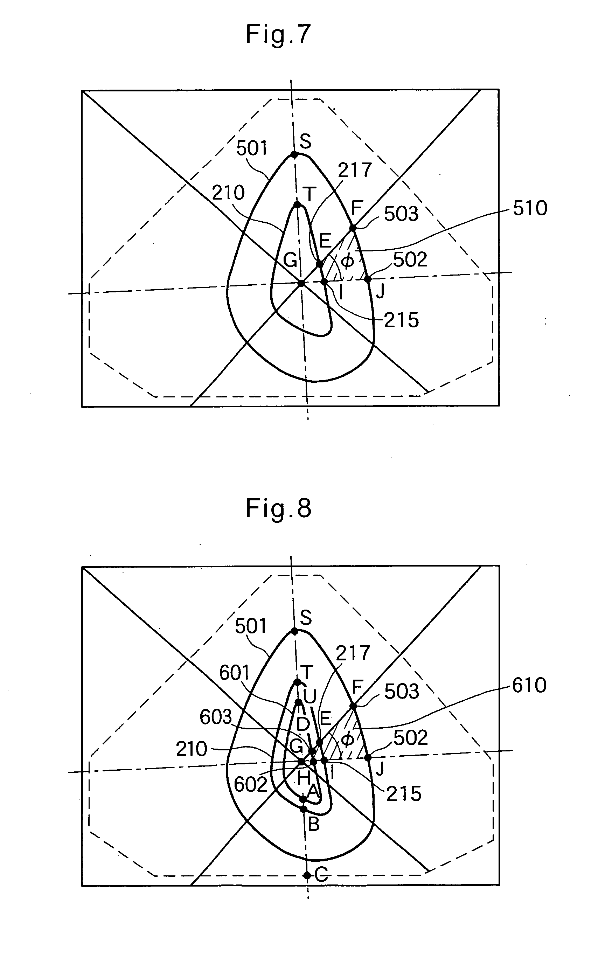

Fig.7 explains an example of the method ② described above. While the area between the initial contour 210 and the bold line 201 is the target of division in the method ① shown in Fig.5, Fig.7 illustrates the case where a boundary 501 is set at a position which is distant from the initial contour 210 by a certain number of pixels (e.g. 50 pixels) in the outward direction, and the doughnut-shaped area between the initial contour 210 and the boundary 501 is divided into eight sub-areas as in the above case. Accordingly, a disclosed area to be formed by connecting the point I215, a point J502, a point F503, and the point E217 (the diagonally shaded area in Fig.7) indicates a sub-area 510 determined by this method.

Fig.8 explains a variation of the method ② described above. While Fig.7 illustrates the case where a target area of division is the doughnut-shaped area between the initial contour 210 and the boundary 501, Fig.8 illustrates the case where a boundary 601 is further set at a position which is distant from the initial contour 210 by a certain number of pixels (e.g. 12 pixels) in the inward direction, and the doughnut-shaped area between the boundary 601 and the boundary 501 is divided into eight sub-areas as in the above case. Accordingly, a disclosed area to be formed by connecting a point H602, the point J502, the point F503, and a point D603 (the diagonally shaded area in Fig.8) indicates a sub-area 610 determined by this method.

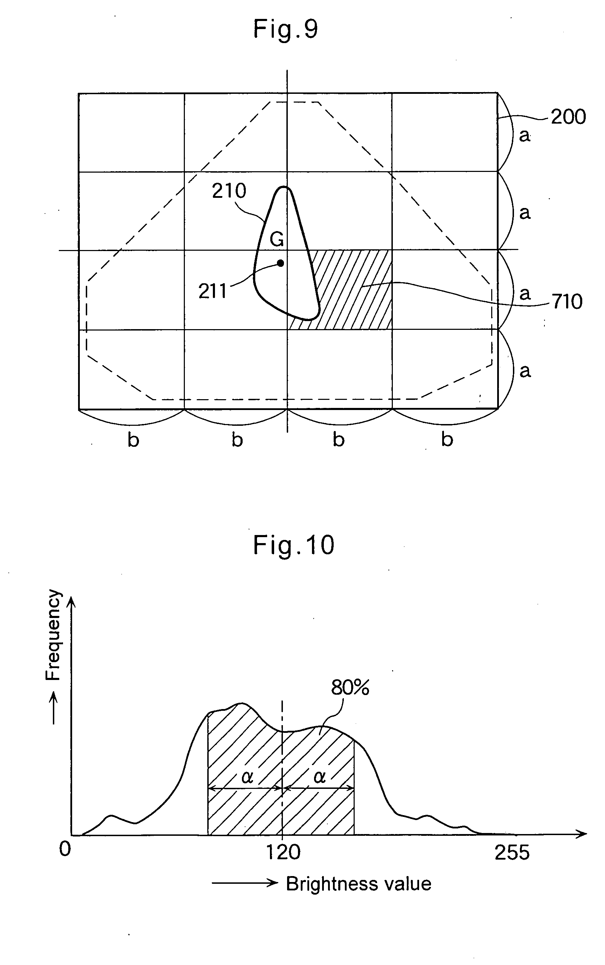

Fig.9 explains an example of the method ③ described above. While ① and ② are methods with which an ultrasound image is divided in a radial pattern with the gravity center G211 of the initial contour 210 as the starting point, Fig.9 illustrates an example case where sub-areas are generated by respectively dividing into quarters the lengths of the X axis and the Y axis within the area which can be displayed on the observation monitor. In this case, the rectangular frame 200 which is the area displayable on the monitor is divided into 16 sub-areas, each of which is equivalent to a rectangular sub-area 710 made up of "a" pixels in the X direction and "b" pixels in the Y direction. Note that division methods illustrated in Figs.5∼9 are only examples and therefore that an arbitrary existing division method (e.g. the straight line connecting the gravity center G211 and the point T212 illustrated in Fig.5 is set as a reference line, and an ultrasound image is divided into equal parts in a counterclockwise direction, each forming an angle of π /3) may be employed by the area dividing unit 103, without being limited to such example methods.

The evaluation value calculating unit 104 calculates an evaluation value used to quantitatively ascertain the quality, characteristics and the like of the ultrasound image for each sub-area divided by the area dividing unit 103. The following are methods for calculating an evaluation value:
(1) Method utilizing brightness values of a sub-area
   With this method, an evaluation value is calculated on the basis of the average value, distribution and the like of the brightness value of each pixel making up the image of a sub-area;
(2) Method utilizing information concerning a contour shape
   With this method, the degree of circularity φ (letting the length of the contour line is "L" and the cross-sectional area is "A", φ=4πA/L**2. If the contour forms a perfect circle, the degree of circularity is 1.0. The more complicated a contour shape is, the smaller a value of the degree of circularity becomes.), acutance or the like calculated on the basis of the contour shape of an object within a sub-area are used as an evaluation value. Note that position-related data such as the distance between the position of the gravity center of the contour of a specified object (i.e. a reference point of the entire ultrasound image) and the reference point of each sub-area is utilized as an evaluation value is some cases. Referring to Fig.9, an explanation is given for an example case where position-related data is used as an evaluation value. First, the gravity center G211 of the initial contour 210 is set as the reference point of the entire ultrasound image. Then, distances from such gravity center G211 and the reference point of each sub-area (in this case, the reference point of each sub-area serves as the gravity center of each sub-area) are set as evaluation values, of which the smallest four values are selected;
(3) Method utilizing edge information
   With this method, an arbitrary edge detection filter (two dimensional differentiation using a filter window) is carried out for a sub-area, and the resulting output is used as an evaluation value (e.g. the amount of differentiation in the directions of X and Y, edge strength);
(4) Method utilizing binarization information
   With this method, binarization is performed for brightness values within a sub-area on a per brightness value basis, using either a specified threshold value or a threshold value to be dynamically determined according to the distribution of brightness values within each sub-area. Then, statistical data or data concerning shape and geography of the binarized data such as its distribution and shape (e.g. acutance) is used as an evaluation value;
(5) Method utilizing the degree of separation between brightness values
   When brightness values are classified into two classes of "0" and "1", "the degree of separation between brightness values" indicates an occupancy ratio of variations between such classes in variations of the all brightness values. If brightness values are perfectly separated into "0" and "1", a separation degree value is 1.0 (maximum value). Note that this method is described in details in "Fukui K. *Contour Extraction Method Based on Separatability of Image Features* (Journal of IEICE D- II vol.J80-D- II, no.6, pp.1406-1414, June 1997)"; and
(6) Method utilizing maximum and minimum brightness values

With this method, the maximum difference to be determined by deducting the minimum brightness value from the maximum brightness value is used as an evaluation value.

The following explains "(1) Method utilizing brightness values of a sub-area" described above. When brightness values are utilized, an evaluation value may be either "the brightness distribution within a sub-area" or "the range width of brightness values occupying 80% of the entire brightness value histogram" that extends from the average value of the brightness values as its center.

A more specific explanation is given for the latter method with reference to Fig.10, which illustrates the case where brightness values of a certain sub-area are distributed between 0∼255 and the brightness average value is "120". In this case, the brightness values in the sub-area are sampled so as to determine "α" when brightness values in 80% of the all pixels (800 pixels if a sub-area is made up of 1000 pixels) satisfy "120±α (α: natural number), and "2α" is used an evaluation value in this case. Note that the above-listed evaluation value calculation methods (1)∼(6) are only examples and therefore that an arbitrary existing expression and image processing may be employed by the evaluation value calculating unit 104 in order to calculate an evaluation value, without being limited to such example methods.

The each area processing unit 105 performs image processing for each sub-area divided by the area dividing unit 103. Image processing here mainly refers to processing for improving image quality of each sub-area. However, such processing may be one that facilitates evaluation processing performed by the evaluation value calculating unit 104 (e.g. normalization for controlling variations in the size of evaluation values among sub-areas), processing intended for enhancing performance of a post-connected apparatus, stabilizing its operations and improving its image quality, and other processing when the image is reconstructed by the image reconstructing unit 107 described later.

The above-mentioned processing for improving image quality includes binarization, contrast controller, bias controller, noise reduction, Morphology process, edge extraction, edge enhancement, some of which, of course, may be combined for use.

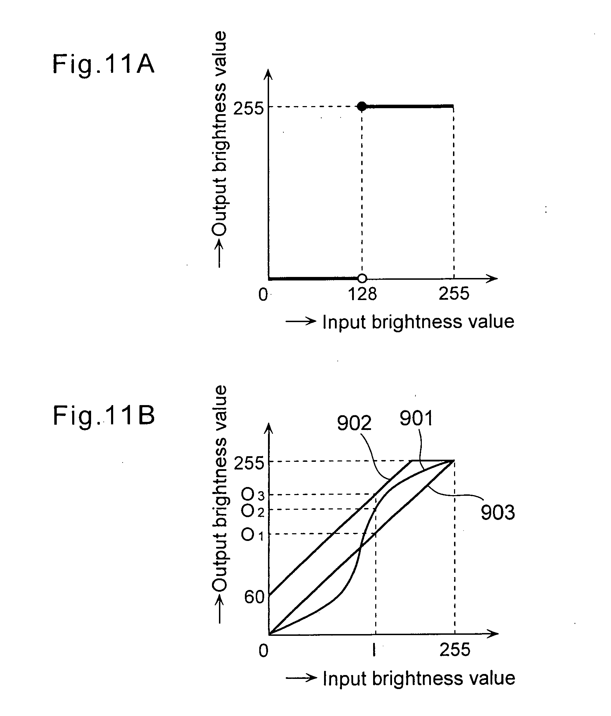

An overview of each process described above is explained with reference to Fig.11.

Fig.11A is a diagram showing values of input brightness and output brightness when binarization is performed. As illustrated in Fig.11A, letting that the threshold value for the input brightness values is "128", an output brightness value varies between 0 and 255 inclusive, when an input brightness value is 128 or over.

Fig.11B is a diagram showing a relationship between input brightness values and output brightness values when contrast controller and bias controller are performed. A curve 901 illustrated in Fig.11B indicates that input brightness values and output brightness values have a nonlinear relationship as a result of contrast controller. A curve 902 illustrated in Fig.11B, on the other hand, shows an output brightness value being outputted which is an input brightness value added (biased) with a certain brightness value, as a result of bias controller. In this case, brightness value to be biased is "60". Note that Fig.11B shows for reference a curve 903 indicating that input brightness values = output brightness values.

An example of noise reduction is a 2D lowpass filter. Morphology process, which is a kind of nonlinear filtering processing, refers to filtering to be performed on the basis of such operations as "dilation" and "erosion" which are intended for extracting features from a given binary image or a contrast image. Note that detailed information for such Morphology process is described in "Kobatake H. *Morphology* (Corona Publishing Co., Ltd.)".

Edge extraction refers to processing for extracting edge indicating area boundaries in an image (e.g. subject and background). There are variations including one using first differential filter and second differential filter.

Edge enhancement refers to processing for enhancing the difference in the contrast level between the edge and other parts in an ultrasound image. Its variations include a method for transforming the distribution of brightness values.

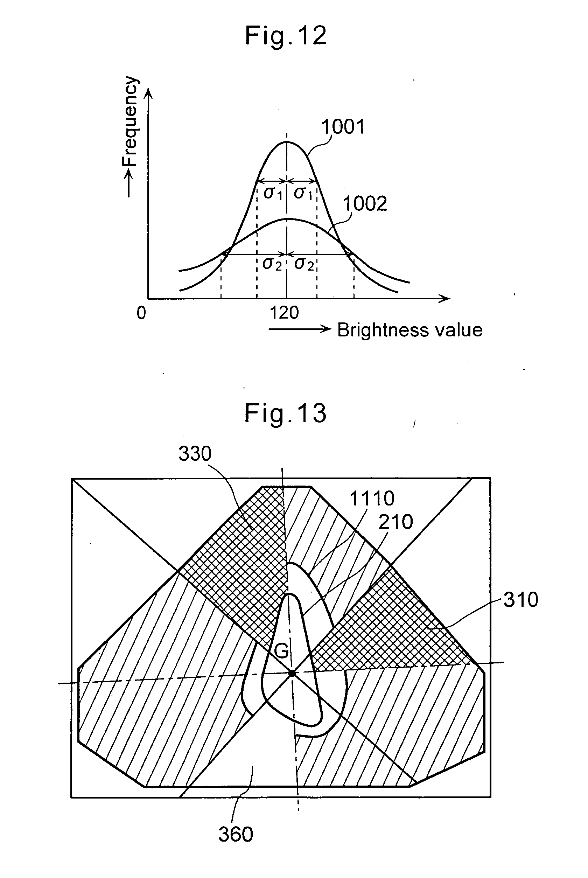

Fig.12 is a diagram showing an example method for transforming the distribution of brightness values. Fig.12 illustrates the case where a curve 1001 indicating that brightness values are centered around the average value (e.g. 120) of the brightness values is transformed into a curve 1002 indicating a less concentrated distribution.

The area selecting unit 106 determines an arbitrary number of sub-areas from the sub-areas divided by the area dividing unit 103. A specified number of sub-areas may be selected from sub-areas with bigger evaluation values calculated by the evaluation value calculating unit 104 in descending order, or from sub-areas with smaller evaluation values in ascending order. The above-mentioned case where "2α" is used as an evaluation value determined on the basis of brightness values is taken as an example. By selecting sub-areas with bigger "2α" in decreasing size order, sub-areas with a clearer contrast (i.e. a wider contrast range) are selected. In contrast, by selecting sub-areas with smaller "2α" in increasing size order, sub-areas with a more unclear contrast (i.e. a narrower contrast range) are selected.

The image reconstructing unit 107 generates new image data by putting together ( i ) image data of the sub-areas which are divided by the area dividing unit 103 and for which image processing is performed by the each area processing unit 105, and (ii) the image data of the ultrasound image generated by the image generating unit 110.

For example, the image reconstructing unit 107 reconstructs the image by using only images within sub-areas specified by the area selecting unit 106 (in this case, one or more sub-areas do not appear as an image). When image processing is performed for each sub-area specified by the. area selecting unit 106, it is also possible for the image reconstructing unit 107 to override an image of each sub-area on the original ultrasound image and to replace an image of each sub-area with the original image.

Next, an explanation is given for the operation of the ultrasonic diagnostic device 10 with the above configuration.

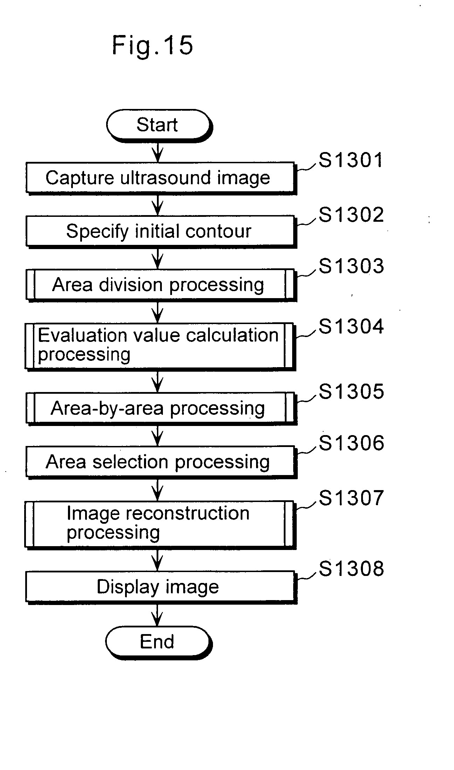

Fig.15 is a flowchart showing an example flow of the entire processing performed by the ultrasonic diagnostic device 10. First, the image generating unit 110 generates an ultrasound image on the basis of an ultrasonic echo received via the ultrasonic search unit 11 and the send/receive unit 12 (S1301).

Next, using an initial contour of a target object which is specified through an operation of the operator on the operation unit 14 or which is automatically extracted by the contour extracting unit 111 (S1302), the area dividing unit 103 divides the ultrasound image displayed on the observation monitor into a plurality of sub-areas (S1303).

Then, the evaluation value calculating unit 104 calculates an evaluation value for each sub-area divided in the above mentioned manner (S1304), and the each area processing unit 105 then performs image processing for such sub-areas on a per sub-area basis (S1305).

Subsequently, when the area selecting unit 106 selects some of the sub-areas in accordance with the calculated evaluation values (S1306), the image reconstructing unit 107 reconstructs the ultrasound image on the observation monitor based on images of the selected sub-areas (S1307). Such reconstructed ultrasound image is then outputted to the data outputting unit 16 to be displayed on the observation monitor or the like.

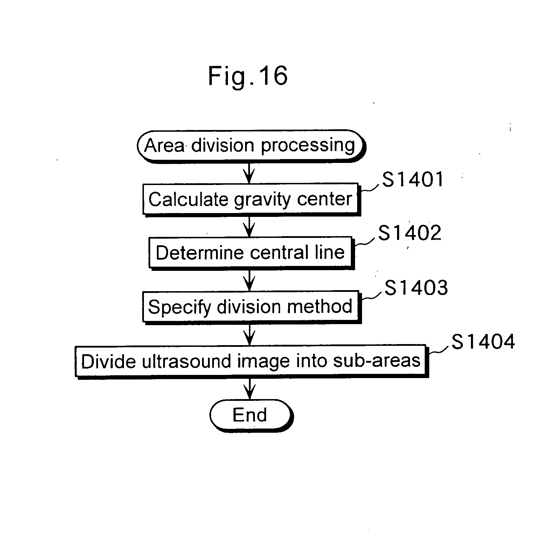

Fig.16 is a flowchart showing an example of "Area division processing (S1303)" illustrated in Fig.15.

First, the area dividing unit 103 calculates a gravity center G of the initial contour specified as above (S1401), so as to determine a central line running on such gravity center G (S1402).

Next, the area dividing unit 103 specifies a division method (e.g. the above mentioned method ①) (S1403), and divides the ultrasound image into a plurality of sub-areas according to the specified division method (S1404).

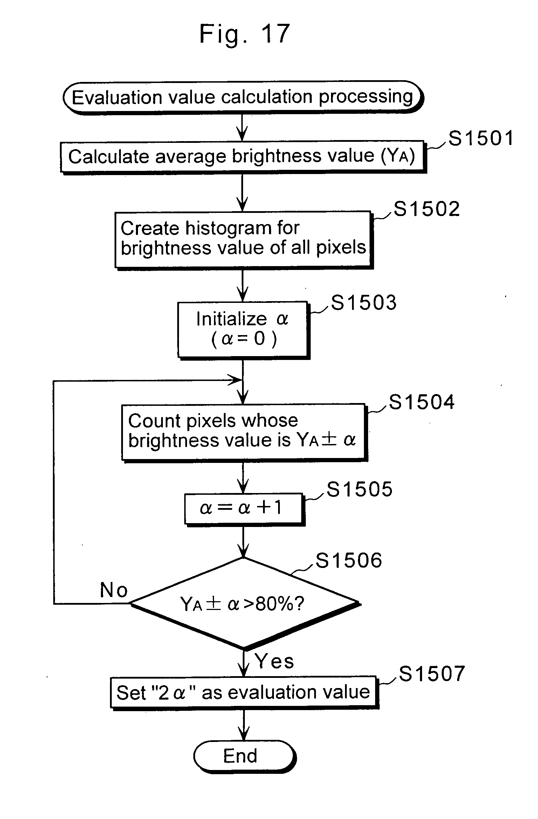

Fig.17 is a flowchart showing an example of "Evaluation value calculation processing" illustrated in Fig.15. Note that Fig.17 illustrates the case where an evaluation value "2α" related to the distribution of brightness values is calculated.

First, the evaluation value calculating unit 104 calculates the average (YA) of brightness values of all pixels included as a target of evaluation value calculation (S1501). Then, the evaluation value calculating unit 104 creates a brightness value histogram that extends from the calculated average value for all the pixels (S1502).

Next, after initializing an increase α (α: natural number) in a brightness value (e.g. α=0) (S1503), the evaluation value calculating unit 104 counts the number of pixels whose brightness value is "YA±α" (S1504). Then, the evaluation value calculating unit 104 updates "α" by adding "1" to it (S1505), and judges whether the number of the counted pixels exceeds 80% of all the pixels, i.e. whether "YA± α>80%" ("α" in this inequality is the pre-updated value) is satisfied or not (S1506). If such condition is satisfied, the evaluation value calculating unit 104 sets "2α" as an evaluation value (S1507).

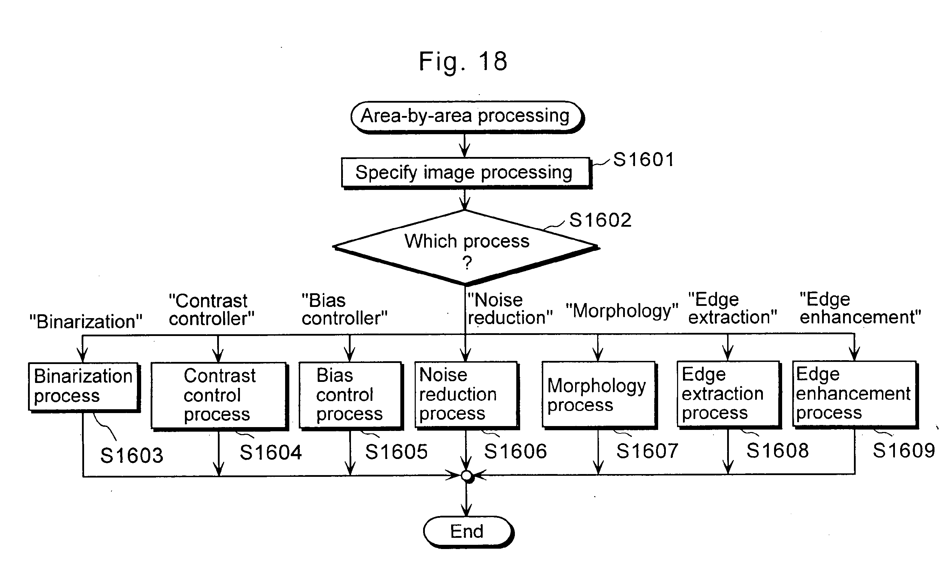

Fig.18 is a detailed flowchart showing "area-by-area processing" illustrated in Fig.15.

First, the each area processing unit 105 accepts the contents of image processing to be carried out from the operator via the operation unit 14 (S1601). In this case, "image processing" includes the following processes: binarization, contrast controller, bias controller, noise reduction, Morphology process, edge extraction and edge enhancement. Then, the each area processing unit 105 executes a specified process (S1602∼S1609). Note that at least one of the above processes (e.g. edge enhancement) may be executed as a default.

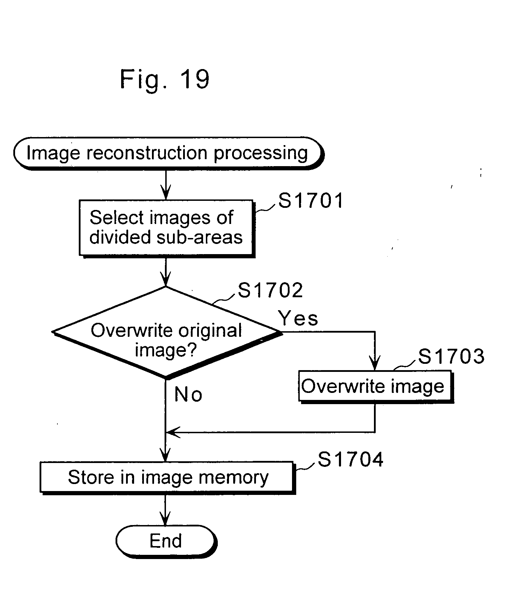

Fig.19 is a flowchart showing the details of "Image reconstruction processing (S1307)" illustrated in Fig.15.

First, the controlling unit 112 accepts via the operation unit 14 an operator's instruction as to the selection of sub-areas to be reconstructed as an image (S1701) and as to whether such sub-areas are overwritten over the original image or not (S1702). If an instruction indicating that overwriting is to be performed is accepted (S1702: Yes), the controlling unit 112 overwrites the ultrasound image generated by the image generating unit 110 with images of the selected sub-areas (S1703), and stores the resulting image in the image memory 101 (S1704).

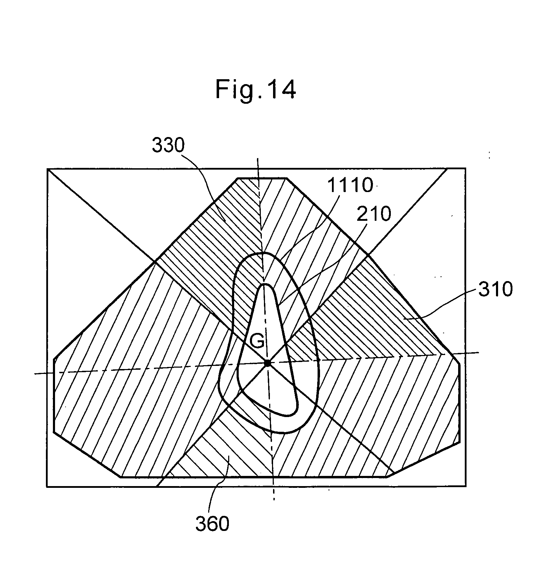

Figs.13 and 14 are diagrams showing, in a simplified manner, the ultrasound image before and after image processing is performed by the each area processing unit 105. As illustrated in Fig.13, of the eight sub-areas divided by the area dividing unit 103, since brightness values of the entire sub-areas 310 and 330 are equally low (i.e. the entire images are blackish), a contour 1110 of an object is partly unclear. In contrast, since brightness values of the image of the sub-area 360 are equally high (i.e. the entire images are whitish), the contour 1110 of the object is partly unclear. Meanwhile, Fig.14 depicts the ultrasound image shown in Fig.13 for which image processing is performed by the each area processing unit 105. As can be seen from Fig.14, image quality of the sub-areas 310, 330 and 360 is improved and the entire contour 1110 of the object has become clear.

As described above, with the ultrasonic diagnostic device according to the present embodiment, it is possible to reliably perform such processing as contour extraction of an object (e.g. LV) even for an image which is partly unclear or blurred.

Note that although the image processing unit 15 according to the present embodiment is configured to be an integral part of the ultrasonic diagnostic device, it is also possible that the image generating unit 110 of the image processing unit 15 is replaced by a data inputting unit capable of accepting image data from outside the device so that the image processing unit 15 can serve as an image processing device having the functions described above.

Note that the image processing unit 15 is also capable of processing image data to be successively inputted in real time (moving image data). In this case, each unit of the image processing unit 15 performs processing on a per frame basis.

As another example, when extracting a contour of an object in an ultrasound image while tracking such object (e.g. when wishing to trace the internal wall of an LV for extracting its contour, while tracking the mitral valve annulus that separates the LV and the left atrium), the operator performs tracking as processing for the inside of sub-areas while performing processing for improving image quality for sub-areas with unclear contours. Then, after such tracking, by notifying from the area selecting unit the position of a sub-area in which the mitral valve annulus exists, it is possible to track and extract its contour in the image for which a conventional ultrasonic diagnostic device cannot perform contour extraction.

"Improving image quality" described in the previous paragraph includes contrast improvement by the use of an histogram equalizer or through noise cut, edge enhancement, or the like, but an arbitrary method may be used without being limited to such examples.

Moreover, "tracking" described above indicates, for example, pattern matching, inter-frame autocorrelation, methods for detecting a motion vector and the like, but an arbitrary method may be used without being limited to such examples.

### (Second Embodiment)

While the first embodiment explains the case where the present invention is applied to an ultrasound image generated by the ultrasonic diagnostic device, the second embodiment describes the case where the present invention is applied to an image generated by an image processing device such as a camera-equipped mobile phone.

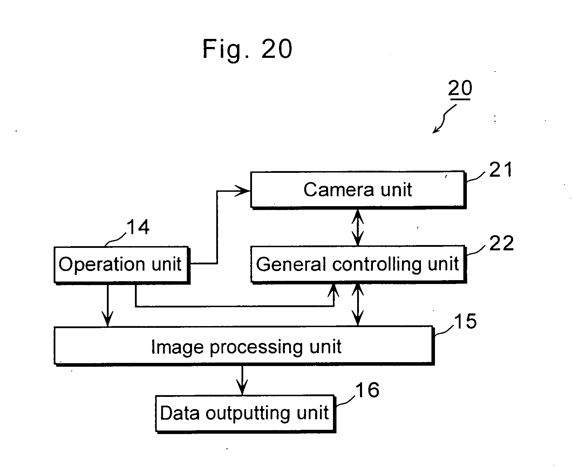

Fig.20 is a block diagram showing a functional configuration of an image processing device 20 according to the present embodiment. The image processing device 20 is capable of performing case-by-case processing for improving image quality even when a part of an image is unclear or blurred. Such image processing device 20 is comprised of a camera unit 21, a general controlling unit 22, the operation unit 14, the image processing unit 15, and the data outputting unit 16 (for convenience of explanation, functions of a general camera-equipped mobile phone such as communication capabilities and memory function are omitted in the image processing device 20).

Note that the image processing device 20 is equivalent to the ultrasonic diagnostic device 10 according to the first embodiment excluding that the image processing device 20 includes the camera unit 21 and the general controlling unit 22 instead of the ultrasonic search unit 11 and the send/receive unit 12 respectively. Note therefore that the following provides explanations focused especially on points that are different from the ultrasonic diagnostic device 10 according to the first embodiment.

The camera unit 21, which includes a CCD and others, is a unit that takes a picture according to an operation of the operator inputted via the operation unit 14 (e.g. photoelectric conversion) and that generates image data.

The general controlling unit 22 has an overall control of the image processing device 20, and includes a CPU, a ROM, a RAM or the like. Furthermore, the general controlling unit 22 receives image data generated by the camera unit 21 to store it to the RAM or the like, and sends to the image processing unit 15 the received image data as it is or image data read out from the RAM or the like, depending on an operator's operation inputted via the operation unit 14. Note that functions of the operation unit 14, the image processing unit 15 and the data outputting unit 16 are equivalent to corresponding units of the ultrasonic diagnostic device 10 according to the first embodiment.

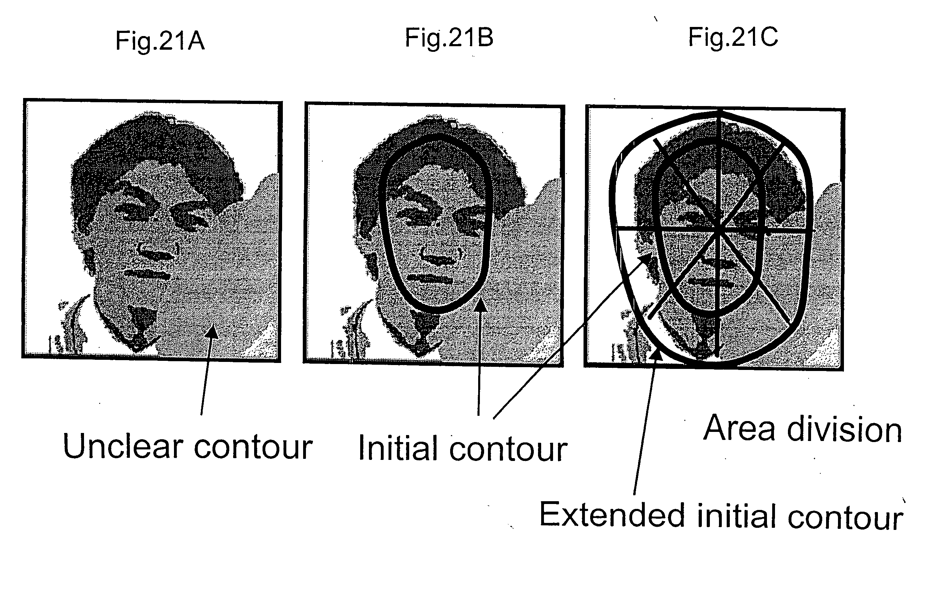

Figs.21A∼21C are diagrams showing an original image taken by a camera-equipped mobile phone or the like until when area division is performed for such original image. Fig.21A is an example original image. As illustrated in Fig.21A, since there is a part in the lower left-hand part of the image that obstructs the subject of the picture (e.g. a part where steam or smoke appears), a part of the face contour is blurred. Fig.21B is a diagram showing the original image in Fig.21A for which an initial contour has been specified by a method equivalent to the one used in the ultrasonic diagnostic device 10 according to the first embodiment.

Fig.21C is a diagram showing the original image in Fig.21B for which area division has been performed through the same method as used in the first embodiment.

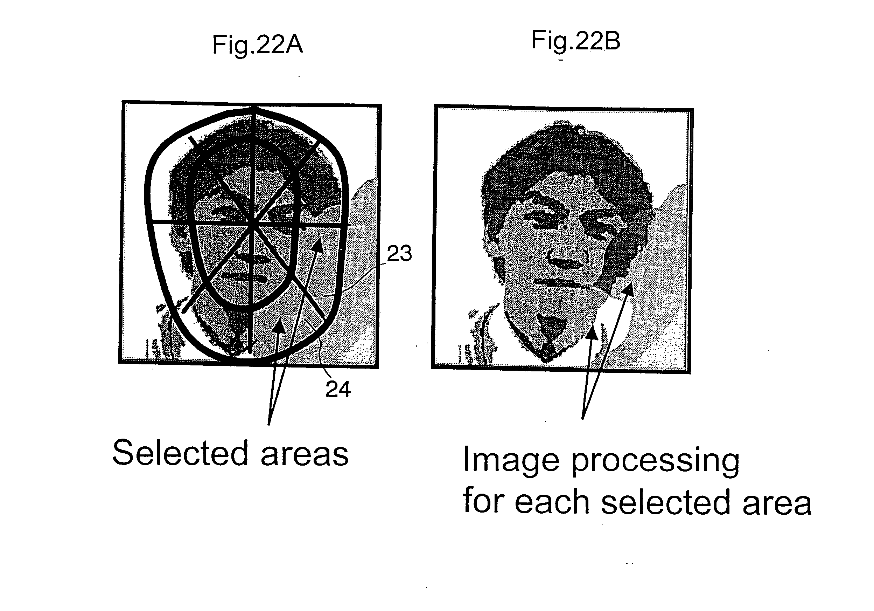

Figs.22A and 22B are diagrams showing the original image in which image processing is performed for sub-areas 23 and 24 selected from among divided sub-areas. Such sub-areas 23 and 24 shown in Fig.22A are two sub-areas selected using the same method presented in the first embodiment. Fig.22B is a diagram showing the original image for which image processing (e.g. contrast controller) has been performed for the sub-areas 23 and 24, as a result of which an improved face contour comes up.

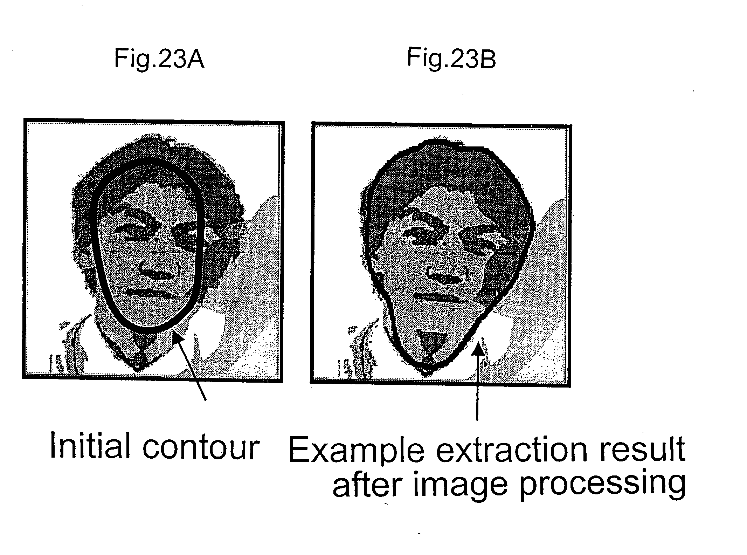

Fig.23A and 23B are diagrams showing that the initial contour is specified again and contour extraction is performed for the image including the sub-areas for which image processing has been performed in the present embodiment. Fig.23A is a diagram showing that the initial contour is specified again for the image including the sub-areas for which image processing has been performed. Fig.23B is a diagram showing a result of more precise contour extraction performed for the image illustrated in Fig.23A for which the initial contour is specified. As shown in Fig.23B, a desirable contour which is approximately the same as the real one is extracted in this case.

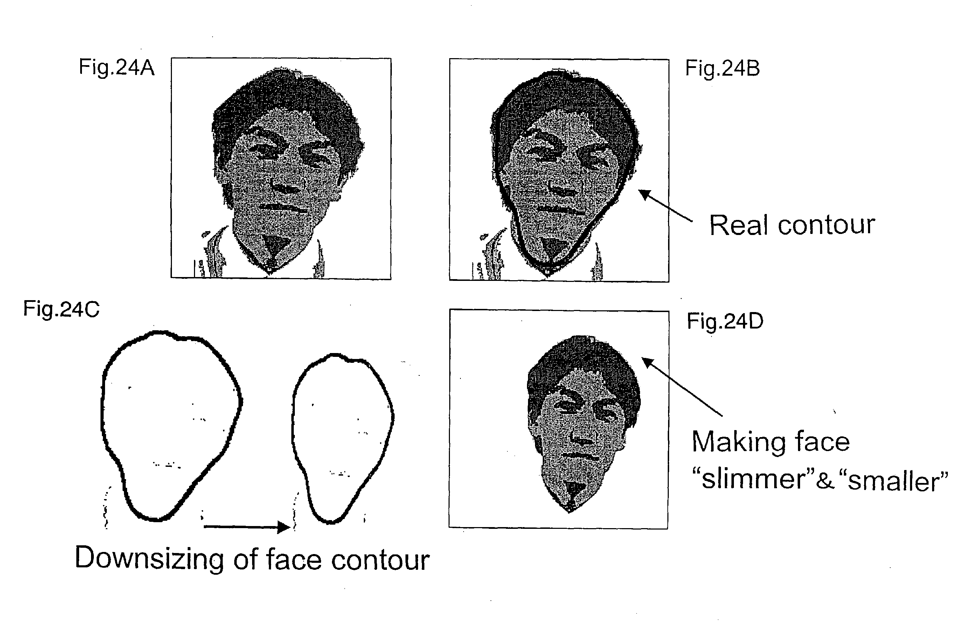

Figs.24A∼24C are diagrams intended to explain an example function added to the image processing device 20. Fig.24B illustrates a result of performing contour extraction for the original image shown in Fig.24A. In this case, the image processing device 20, as illustrated in Fig.24D, is capable of making the face contour "smaller" and "slimmer" on the basis of the extracted contour. A face contour can be made "smaller" or "slimmer", as shown in Fig.24C for example, by setting the scaling factor for the horizontal size (e.g. 0.7) lower than that for the vertical size (e.g. 0.9).

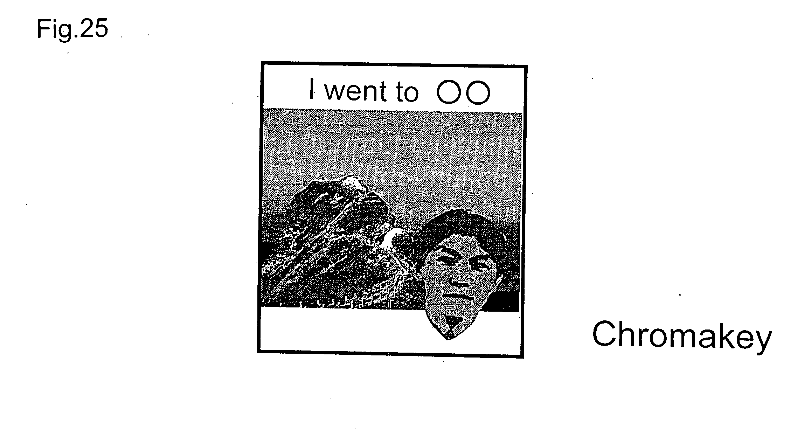

Fig.25 is a diagram intended to explain another example function added to the image processing device 20. As illustrated in Fig.25, the image processing device 20 is capable of extracting a part of the image on the basis of the extracted contour and combining such extracted image with another image (e.g. a scenic image) so as to generate a new image (e.g. chromakey)

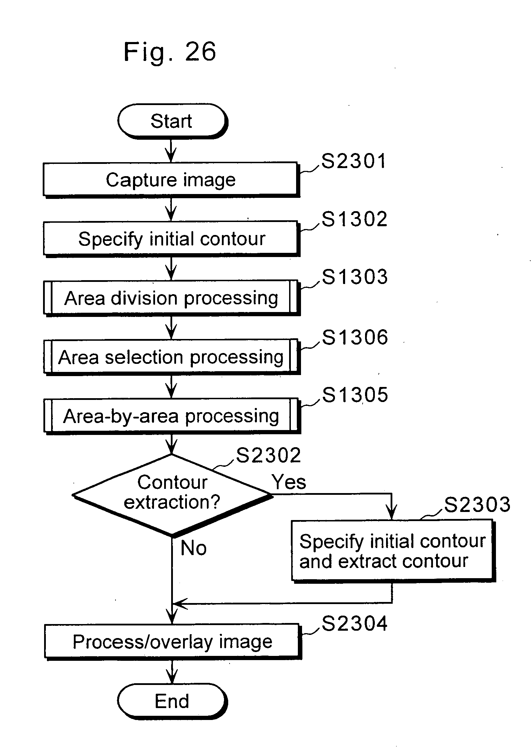

Fig.26 is a flowchart showing an overall flow of processing performed by the image processing device 20. First, the image generating unit 15 generates an image on the basis of image data received via the camera unit 21 and the general controlling unit 22 (S2301).

Next, the general controlling unit 22 identifies an initial contour of a subject according to an operator' operation or through automatic extraction (S1302). Subsequently, the area dividing unit 103 divides the image shown on the display into a plurality of sub-areas (S1303). Then, the general controlling unit 22 accepts the selection of sub-areas from the operator (S1306), and gives an instruction to the each area processing unit 105 to perform image processing on a per sub-area basis (S1305).

Then, upon the receipt of an instruction from the operator indicating that contour extraction is performed again (S2302: Yes), the general controlling unit 22 gives an instruction to each unit so as to have each unit specify the initial contour and extract the contour of the subject as described above (S2303).

Furthermore, the image processing unit 15 performs processing and overlay for the obtained image at the instruction of the general controlling until 22 (S2304).

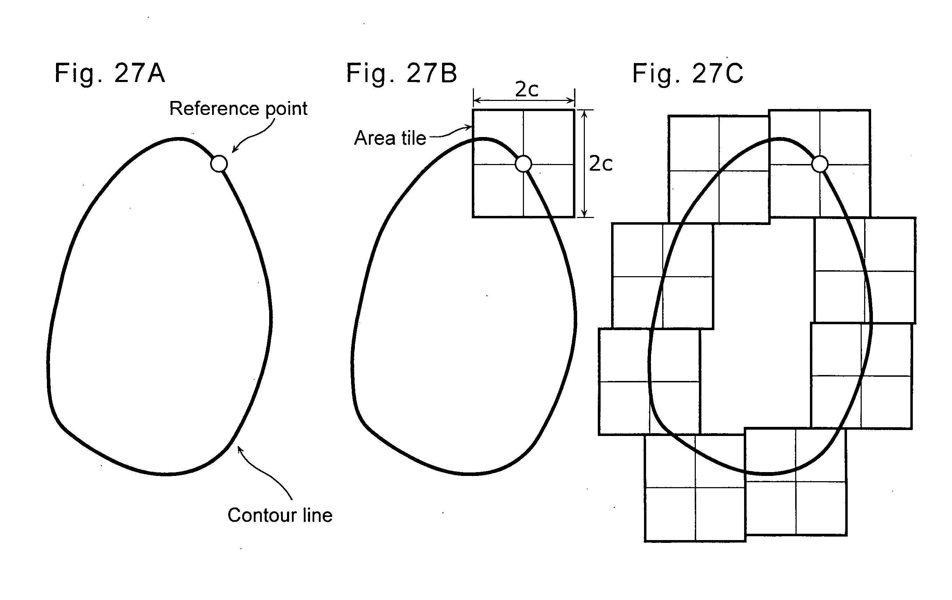

Note that although the area division methods employed by the sub-area dividing unit are illustrated in Figs.5∼9 and Fig.21 in the first and the second embodiments, it should be understood that the present invention are not restricted to such methods. As illustrated in Figs.27A and 27B, for example, an image including the contour line may be divided in a manner in which an area tile which is "2C" on a side is defined with the reference point as its starting point and other area tiles are placed in the same manner. Accordingly, as illustrated in Fig.27C, the image can be divided in accordance with eight area tiles by tracing the contour line. In this case, the area tiles are placed with their center being on the contour line.

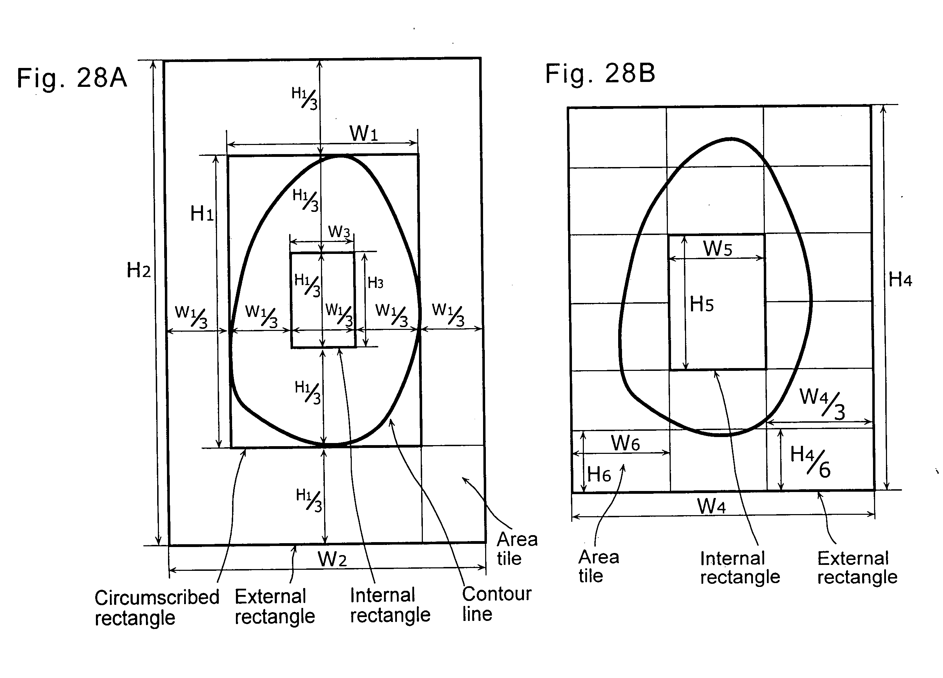

Another division method is illustrated in Figs.28A and 28B, in which the area between an external rectangle and an internal rectangle is divided into sub-areas (area tiles).

In Fig.28A, a circumscribed rectangle (width W1, length H1) circumscribing the contour line is defined first. Then, based on the shape of such circumscribed rectangle, an external rectangle (width W2, length H2) and an internal rectangle (width W3, length H3) are defined. More specifically, rectangles which satisfy W2=5W1/3, H2=5H1/3, W3=W1/3, and H3=H1/3 are satisfied.

In Fig.28B, an external rectangle (width W4, length H4) which internally includes the contour line is defined first, and then an internal rectangle (width W5, length H5) is defined inside the contour line. More specifically, rectangles which satisfy W5=W4/3 and H5=H3/3 are defined. Furthermore, the area between such external rectangle and such internal rectangle is divided in accordance with area tiles (width W6, length H6). To be more specific, area tiles each of which satisfies W6=W4/3 and H6=H4/6 are defined.

Note that values of c (see Fig.27B), W1∼W6, and H1∼H6 may be changed to other values according to an instruction from the operator accepted via the operation unit 14 and that such changed values are used in corresponding methods for area division. Also note that the above dimensions are just examples and therefore that other dimensions are employed for image division.

As described above, with the image processing device according to the present embodiment, it is possible to extract from an image the contour of a face or the like whose contour appears unclear or blurred (i.e. improve image quality) by the use of the same method employed in the ultrasonic diagnostic device according to the first embodiment. Note that although the explanation provided in the present embodiment focuses on a face, it should be understood that the present invention is also applicable to the extraction of a contour of an arbitrary object.

## Claims

1. An image processing device comprising:
an image acquiring unit operable to acquire image data;
an area dividing unit operable to divide an image represented by the acquired image data into a plurality of sub-areas;
an area selecting unit operable to make a selection of one or more of the sub-areas; and
an each area processing unit operable to perform image processing for each of said one or more selected sub-areas.

2. The image processing device according to Claim1,
wherein the image data relates to an ultrasound image that is generated on the basis of an ultrasonic echo, and
the image acquiring unit acquires the image, data from an ultrasonic diagnostic device.

3. The image processing device according to Claim 1,
wherein the image data relates to an image that is taken by the use of a charge coupled device, and
the image acquiring unit acquires the image data from a CCD camera.

4. The image processing device according to Claim1,
wherein the area selecting unit makes the selection according to a distance between a reference point of the entire image and a reference point of each of the divided sub-areas.

5. The image processing device according to Claim1 further comprises an evaluation value calculating unit operable to calculate evaluation values, each indicating image clarity of each of the divided sub-areas,
wherein the area selecting unit makes the selection on the basis of the calculated evaluation values.

6. The image processing device according to Claims,
wherein the area selecting unit makes a selection from the sub-areas in decreasing order of unclarity indicated by the evaluation values.

7. The image processing device according to Claims,
wherein the evaluation value calculating unit calculates the evaluation values using at least one of the following sub-area information: brightness value information; shape information; edge information; binarization information; separation degree information; and maximum/minimum brightness value information.

8. The image processing device according to Claim6,
wherein the each area processing unit performs at least one of the following processes as the image processing: edge extraction process; edge enhancement process; binarization process; contrast control process; bias control process; noise reduction process; and Morphology process.

9. The image processing device according to Claim8,
wherein the area dividing unit makes the division of the image by dividing the image into a specified number of equal parts in directions of an X axis and a Y axis respectively.

10. The image processing device according to Claim8,
wherein the area dividing unit includes:
a contour information acquiring unit operable to acquire contour information indicating a contour of an object in the image;
a gravity center calculating unit operable to calculate a gravity center of an image specified by the contour indicated by the acquired contour information; and
a reference point identifying unit operable to identify a reference point on the contour, and
the area dividing unit makes the division of the image on the basis of a straight line connecting the gravity center and the reference point, by dividing the image starting from the gravity center in a radial pattern at a specified angle.

11. The image processing device according to Claim8,
wherein the area dividing unit includes:
a contour information acquiring unit operable to acquire contour information indicating a contour of an object in the image;
a circumscribed rectangle setting unit operable to set a rectangle circumscribing the contour;
an internal rectangle setting unit operable to set an internal rectangle inside the circumscribed rectangle;
an external rectangle setting unit operable to set an external rectangle outside the circumscribed rectangle; and
a sub-area dividing unit operable to divide an area between the internal rectangle and the external rectangle on the basis of the circumscribed rectangle.

12. The image processing device according to Claim8,
wherein the area dividing unit includes:
a contour information acquiring unit operable to acquire contour information indicating a contour of an object in the image;
a reference point identifying unit operable to identify a reference point on the contour, and
a sub-area placing unit operable to place, on the contour, a sub-area in a specified shape having the reference point as a center, and
the image includes the placed sub-area.

13. The image processing device according to Claim 12,
wherein the area dividing unit further includes a sub-area changing unit operable to accept an instruction for changing a shape of the sub-areas.

14. The image processing device according to one of Claims9∼1 2 further comprises an image reconstructing unit operable to reconstruct the image using an image of said one or more selected sub-areas for which the image processing is performed.

15. The image processing device according to Claim14,
wherein the image reconstructing unit replaces, with the image of said one or more selected sub-areas for which the image processing is performed, a corresponding image of the sub-areas in the acquired image.

16. The image processing device according to Claim15 further comprises a contour re-extracting unit operable to acquire contour information indicating a contour of the object in the replaced image.

17. An image processing method including:
an image acquiring step for acquiring image data;
an area dividing step for dividing an image represented by the acquired image data into a plurality of sub-areas;
an area selecting step for making a selection of one or more of the sub-areas; and
an each area processing step for performing specific image processing for each of said one or more selected sub-areas.

18. An image processing method including:
an image acquiring step for acquiring image data;
an area dividing step for dividing an image represented by the acquired image data into a plurality of sub-areas;
an evaluation value calculating step for calculating evaluation values, each indicating image clarity of each of the divided sub-areas;
an area selecting step for making a selection of one or more of the sub-areas on the basis of the calculated evaluation values; and
an each area processing step for performing specific image processing for each of said one or more selected sub-areas.

19. A program for an image processing device including:
an image acquiring step for acquiring image data;
an area dividing step for dividing an image represented by the acquired image data into a plurality of sub-areas;
an area selecting step for making a selection of one or more of the sub-areas; and
an each area processing step for performing specific image processing for each of said one or more selected sub-areas.

20. A program for an image processing device including:
an image acquiring step for acquiring image data;
an area dividing step for dividing an image represented by the acquired image data into a plurality of sub-areas;
an evaluation value calculating step for calculating evaluation values, each indicating image clarity of each of the divided sub-areas;
an area selecting step for making a selection of one or more of the sub-areas on the basis of the calculated evaluation values; and
an each area processing step for performing specific image processing for each of said one or more selected sub-areas.

21. An ultrasonic diagnostic device that displays an ultrasound image of an object subject to examination generated on the basis of a reflection of ultrasound, the ultrasonic diagnostic device comprising:
an image acquiring unit operable to acquire image data;
an area dividing unit operable to divide an ultrasound image represented by the acquired image data into a plurality of sub-areas;
an area selecting unit operable to make a selection of one or more of the sub-areas;
an each area processing unit operable to perform specific image processing for each of said one or more selected sub-areas; and
a displaying unit operable to display an image of said one or more selected sub-areas for which the image processing is performed.

22. An ultrasonic diagnostic device that displays an ultrasound image of an object subject to examination generated on the basis of a reflection of ultrasound, the ultrasonic diagnostic device comprising:
an image acquiring unit operable to acquire image data;
an area dividing unit operable to divide an ultrasound image represented by the acquired image data into a plurality of sub-areas;
an evaluation value calculating unit operable to calculate evaluation values, each indicating image clarity of each of the divided sub-areas;
an area selecting unit operable to make a selection of one or more of the sub-areas on the basis of the calculated evaluation values;
an each area processing unit operable to perform specific image processing for each of said one or more selected sub-areas;
an image reconstructing unit operable to reconstruct the ultrasound image of the examined object using an image of said one or more selected sub-areas for which the image processing is performed; and
a displaying unit operable to display the reconstructed ultrasound image.

23. A program for an ultrasonic diagnostic device that displays an ultrasound image of an object subject to examination generated on the basis of a reflection of ultrasound, the program having a computer execute the following steps:
an image acquiring step for acquiring image data;
an area dividing step for dividing an ultrasound image represented by the acquired image data into a plurality of sub-areas;
an area selecting step for making a selection of one or more of the sub-areas;
an each area processing step for performing specific image processing for each of said one or more selected sub-areas; and
a displaying step for displaying an image of said one or more selected sub-areas for which the image processing is performed.

24. A program for an ultrasonic diagnostic device that displays an ultrasound image of an object subject to examination generated on the basis of a reflection of ultrasound, the program having a computer execute the following steps:
an image acquiring step for acquiring image data;
an area dividing step for dividing an ultrasound image represented by the acquired image data into a plurality of sub-areas;
an evaluation value calculating step for calculating evaluation values, each indicating image clarity of each of the divided sub-areas;
an area selecting step for making a selection of one or more of the sub-areas on the basis of the calculated evaluation values;
an each area processing step for performing specific image processing for each of said one or more selected sub-areas;
an image reconstructing step for reconstructing the ultrasound image of the examined object using an image of said one or more selected sub-areas for which the image processing is performed; and
a displaying step for displaying the reconstructed ultrasound image.
